# EUROPÄISCHE PATENTANMELDUNG

(11) **EP 4 603 176 A1**
(43) Veröffentlichungstag der Anmeldung: **20.08.2025**
(21) Anmeldenummer: 24157740.2
(22) Anmeldetag: 15.02.2024
(51) Int. Cl.: B01F 31/441, B01F 33/501, B01F 35/71, B01F 35/75, A61B 17/88, B01F 101/20

(54) **KNOCHENZEMENT-MISCHSYSTEM MIT VERBESSERTEM MONOMERFLÜSSIGKEITSTRANSFER**

(71) Anmelder: Heraeus Medical GmbH, 61273 Wehrheim (DE)
(72) Erfinder: Dr. Kluge, Thomas, 61273 Wehrheim (DE); Dr. Vogt, Sebastian, 61273 Wehrheim (DE)
(74) Vertreter: Heraeus IP

(57) **Zusammenfassung**

Die vorliegende Erfindung betrifft eine Vorrichtung zum Mischen von Knochenzement, aufweisend eine Kartusche 101 zur Aufnahme eines Knochenzementpulvers; einen Behälter 102 zur Aufnahme einer Monomerflüssigkeit 118, wobei der Behälter fluidleitend mit der Kartusche 101 verbunden ist; einen an einem proximalen Ende 103 der Kartusche angeordneten Kartuschenkopf 104, wobei der Kartuschenkopf eine Durchführung 105 aufweist; ein Einleitungsrohr 106, wobei das Einleitungsrohr eine Längsachse 107 aufweist; und einen trichterförmigen Auslass 108, wobei der trichterförmige Auslass eine Trichterinnenfläche 109 aufweist, die zumindest teilweise in einem Winkel von nicht mehr als 60° zur Längsachse des Einleitungsrohrs angeordnet ist; wobei die Vorrichtung ausgestaltet und eingerichtet ist, eine Monomerflüssigkeit aus dem Behälter durch freien Gravitationsfluss über den trichterförmigen Auslass und das Einleitungsrohr in die Kartusche zu überführen.

## Beschreibung

### GEBIET DER ERFINDUNG

Die vorliegende Erfindung betrifft das Gebiet der Medizintechnik, insbesondere Vorrichtungen zur Herstellung eines PMMA-Knochenzements und zur Lagerung von Komponenten davon.

### STAND DER TECHNIK

Polymethylmethacrylat-Knochenzemente gehen auf die grundlegenden Arbeiten von CHARNLEY zurück. Polymethylmethacrylat-Knochenzemente bestehen üblicherweise aus einer flüssigen Monomerkomponente und einer Pulverkomponente. Die Monomerkomponente kann beispielsweise das Monomer Methylmethacrylat und einen darin gelösten Aktivator (z.B. N,N-Dimethyl-p-toluidin) enthalten. Die Pulverkomponente, auch als Knochenzementpulver bezeichnet, kann ein oder mehrere Polymere aufweisen, die auf Basis von Methylmethacrylat und Comonomeren, wie Styren, Methylacrylat oder ähnlichen Monomeren durch Polymerisation, vorzugsweise Suspensionspolymerisation, herstellbar sein können. Die Pulverkomponente kann weiterhin einen Röntgenopaker und einen Initiator wie z.B. Dibenzoylperoxid aufweisen. Beim Vermischen der Pulverkomponente mit der Monomerkomponente kann durch Quellung der Polymere der Pulverkomponente im Methylmethacrylat ein plastisch verformbarer Teig entstehen, der eigentliche Knochenzementteig. Beim Vermischen der Pulverkomponente mit der Monomerkomponente reagiert beispielsweise der Aktivator N,N-Dimethyl-p-toluidin mit Dibenzoylperoxid unter Bildung von Radikalen. Die gebildeten Radikale können die radikalische Polymerisation des Methylmethacrylates initiieren. Mit fortschreitender Polymerisation des Methylmethacrylates erhöht sich die Viskosität des Zementteigs, bis dieser erstarrt. Polymethylmethacrylat-Knochenzemente können in geeigneten Mischbechern mit Hilfe von Spateln durch Vermischen der Pulverkomponente mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen "Full-Prepacked-Mischsysteme" sind in den Patentdokumenten EP0380867B1, EP0796653B1, EP0692229B1, US5997544A, US6709149B1, DE69812726T2 und US5588745A, WO9416951A1, DE19718648A1, EP1741413B1, EP3054880B1, DE102009031178B3, US8662736B2, EP2269718B1, EP2281532B1 und EP3093067B1 beschrieben.

Bei Full-Prepacked-Mischsystemen stellen sich einige Herausforderungen. Wünschenswert ist der sichere, reproduzierbare Transfer der Monomerflüssigkeit in das Polymethylmethacrylat-Knochenzementpulver und die Vermeidung einer unkontrollierten Mischung der Komponenten während der Lagerung. Vorteilhaft ist, wenn das Zementpulver während des Transports und der Lagerung des Mischsystems nicht in das Leitungsmittel zum Transfer der Monomerflüssigkeit eindringen kann. Ansonsten kann das Leitungsmittel beim Monomertransfer durch Quellung und Verklebung verstopfen. Eine Blockierung des Leitungsmittels durch gequollenen, klebenden Knochenzement könnte den Monomerflüssigkeitstransfer unterbrechen und die Vermischung der Zementkomponenten im vorbestimmten Mischungsverhältnis verhindern.

Bei den Full-Prepacked-Mischsystemen gemäß EP0796653, US6709149, EP1741413, EP3054880, EP2269718B1 und EP2281532B1 erfolgt der Monomertransfer durch Anlegen eines externen Vakuums an die Kartusche über einen Vakkumanschluss und einer porösen Platte. Das bedeutet, dass die Monomerflüssigkeit durch das angelegte Vakuum in die Kartusche gesaugt wird. Bei dem in der EP3093067B1 beschriebenen Mischsystem kann entweder ein Transfer der Monomerflüssigkeit in die Kartusche durch Vakuumeinwirkung, oder alternativ ein Drucktransfer der Monomerflüssigkeit durch einen in das Mischsystem integrierten, manuell bedienbaren Pumpkolben erfolgen.

Die Blockierung des Leitungsmittels zum Transfer der Monomerflüssigkeit wird gemäß EP2281532B1 dadurch vermieden, dass ein für die Monomerflüssigkeit permeables Netz oder eine Porenscheibe zum Blockieren der Zementpulverpartikel genutzt wird, wobei das Netz oder die Porenscheibe Bestandteil einer speziellen Düse sind, die einen scharfen Monomerflüssigkeitsstrahl beim Transfer erzeugt, der gequollene Zementpartikel verdrängt, so dass ein vollständiger Monomerflüssigkeitstransfer in das Knochenzementpulver möglich ist.

Beim dem in EP1741413B1 und EP3054880B1 beschriebenen Mischsystem werden Gummikappen zur Verhinderung der Migration von Partikeln der Pulverkomponente eingesetzt, die auf hohlen Stechdornen platziert sind. Vor dem Monomertransfer werden diese Gummikappen von den Hohldornen durchstochen und sind dann für den Monomerflüssigkeitstransfer geöffnet.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen. Beispielsweise liefert die Erfindung eine Vorrichtung zum Lagern und Mischen von Knochenzement, die eine einfache und zuverlässige Mischung einer Monomerflüssigkeit mit einer Pulverkomponente eines PMMA-Knochenzements durch Gravitationsfluss erlaubt, um einen gebrauchsfertigen PMMA-Knochenzementteig daraus herzustellen.

Diese Aufgaben werden gelöst durch die hierin beschriebenen Verfahren, Vorrichtungen, Kits und medizinischen Verwendungen, insbesondere denjenigen, die in den Patentansprüchen beschrieben sind.

Bevorzugte Ausführungsformen der Erfindung werden nachfolgend beschrieben.

Eine erste Ausführungsform betrifft eine Vorrichtung Vorrichtung zum Mischen von Knochenzement, aufweisend
eine Kartusche zur Aufnahme eines PMMA-Knochenzementpulvers;
einen Behälter zur Aufnahme einer Monomerflüssigkeit, wobei der Behälter fluidleitend mit der Kartusche verbunden ist;
einen an einem proximalen Ende der Kartusche angeordneten Kartuschenkopf, wobei der Kartuschenkopf eine Durchführung aufweist;
ein Einleitungsrohr, wobei das Einleitungsrohr eine Längsachse aufweist;
und einen trichterförmigen Auslass, wobei der trichterförmige Auslass eine Trichterinnenfläche aufweist, die zumindest teilweise in einem Winkel von nicht mehr als 60° zur Längsachse des Einleitungsrohrs angeordnet ist;
wobei die Vorrichtung ausgestaltet und eingerichtet ist, eine Monomerflüssigkeit aus dem Behälter durch freien Gravitationsfluss über den trichterförmigen Auslass und das Einleitungsrohr in die Kartusche zu überführen.

Eine zweite Ausführungsform betrifft Vorrichtung gemäß der ersten Ausführungsform, wobei das Einleitungsrohr einen Innendurchmesser **i** von 4,0 mm bis 10,0 mm aufweist.

Eine dritte Ausführungsform betrifft eine Vorrichtung gemäß einer der vorangehenden Ausführungsformen, wobei das Einleitungsrohr eine Länge **L** und einen Innendurchmesser **i** aufweist, und wobei das Verhältnis **L** : **i** von Länge **L** zu Innendurchmesser **i** in einem Bereich von **"L** : **i = 1** : **10"** bis **"L** : **i = 1** : **3,5"** liegt.

Eine vierte Ausführungsform betrifft eine Vorrichtung gemäß einer der vorangehenden Ausführungsformen, wobei der trichterförmige Auslass weiterhin ausgestaltet und angeordnet ist, ein Abfließen eines PMMA-Knochenzementpulvers durch freien Gravitationsfluss in die Kartusche zu ermöglichen.

Eine fünfte Ausführungsform betrifft eine Vorrichtung gemäß einer der vorangehenden Ausführungsformen, welche weiterhin ein Mittel zur Eliminierung von PMMA-Knochenzementpulverbrücken in dem Auslass aufweist, wobei das Mittel zur Eliminierung von PMMA-Knochenzementpulverbrücken bevorzugt eine Kugel ist, wobei die Kugel weiter bevorzugt beweglich in dem trichterförmigen Auslass angeordnet ist.

Eine sechste Ausführungsform betrifft eine Vorrichtung gemäß einer der vorangehenden Ausführungsformen, wobei die Trichterinnenfläche über mindestens 50% ihrer gesamten Fläche einen Winkel von nicht mehr als 60°, bevorzugt nicht mehr als 55°, weiter bevorzugt einen Winkel von 21° bis 50°, zur Längsachse der Einleitungsrohrs bildet.

Eine siebte Ausführungsform betrifft eine Vorrichtung gemäß einer der vorangehenden Ausführungsformen, wobei die Kartusche ein PMMA-Knochenzementpulver aufweist, und/oder wobei der Behälter eine Monomerflüssigkeit aufweist.

Eine achte Ausführungsform betrifft eine Vorrichtung gemäß einer der vorangehenden Ausführungsformen, wobei der trichterförmige Auslass ein verjüngtes Ende aufweist, welches unmittelbar fluidleitend mit dem Einleitungsrohr verbunden ist.

Eine neunte Ausführungsform betrifft eine Vorrichtung gemäß einer der vorangehenden Ausführungsformen, wobei der Behälter weiterhin einen für eine Monomerflüssigkeit durchlässigen Filter aufweist, wobei der Filter bevorzugt angeordnet und eingerichtet ist, ein Eindringen von Glassplittern aus dem Behälter in die Kartusche zu verhindern, und wobei der Filter an einer Seite des Filters bevorzugt eine Oberfläche von mind. 5 cm², weiter bevorzugt mind. 10 cm², noch weiter bevorzugt 15 cm² aufweist.

Eine zehnte Ausführungsform betrifft eine Vorrichtung gemäß einer der vorangehenden Ausführungsformen, wobei ein Innenraum des trichterförmigen Auslass und ein Innenraum der Kartusche gemeinsam einen kontinuierlichen Hohlraum bilden.

Eine elfte Ausführungsform betrifft eine Vorrichtung gemäß einer der vorangehenden Ausführungsformen, wobei der trichterförmige Auslass ausgestaltet und angeordnet ist, die Ausbildung eines Meniskus zu verhindern, wenn eine Monomerflüssigkeit aus dem Behälter durch freien Gravitationsfluss über den trichterförmigen Auslass in die Kartusche überführt wird.

Eine zwölfte Ausführungsform betrifft eine Vorrichtung gemäß einer der vorangehenden Ausführungsformen, weiterhin aufweisend einen Kolben zur Abgabe von Knochenzement aus der Vorrichtung, wobei der Kolben zumindest teilweise in der Kartusche angeordnet ist und die Kartusche nach außen begrenzt, und wobei der Kolben bevorzugt eine lösbare Verriegelung aufweist.

Eine dreizehnte Ausführungsform betrifft eine Vorrichtung gemäß einer der vorangehenden Ausführungsformen, weiterhin aufweisend einen Mischstab, wobei der Mischstab bevorzugt ein verschließbares Austragsrohr aufweist, wobei das Austragsrohr bevorzugt ausgebildet und eingerichtet zur Abgabe eines in der Vorrichtung gemischten Knochenzementteigs aus der Vorrichtung ist.

Eine vierzehnte Ausführungsform betrifft eine Vorrichtung gemäß einer der vorangehenden Ausführungsformen, wobei der Behälter weiterhin einen Träger zur Aufnahme einer Ampulle aufweist.

Eine fünfzehnte Ausführungsform betrifft eine Vorrichtung gemäß einer der vorangehenden Ausführungsformen, wobei der Behälter weiterhin ein Mittel zur Öffnung eines Folienbeutels oder einer Glasampulle innerhalb der Vorrichtung aufweist.

Eine sechzehnte Ausführungsform betrifft eine Vorrichtung gemäß einer der vorangehenden Ausführungsformen, wobei die Vorrichtung ausgebildet und eingerichtet ist, eine Entlüftung der Kartusche durch den trichterförmigen Auslass zu ermöglichen, während eine Monomerflüssigkeit aus dem Behälter durch freien Gravitationsfluss über den trichterförmigen Auslass und das Einleitungsrohr in die Kartusche überführt wird.

Ein weiterer Aspekt betrifft ein Verfahren zur Herstellung eines Knochenzementteigs, wobei das Verfahren die nachfolgenden Schritte aufweist,
Bereitstellen einer Vorrichtung nach einer der vorangehenden Ausführungsformen; gegebenenfalls Einbringen einer Monomerflüssigkeit in den Behälter;
gegebenenfalls Einbringen eines PMMA-Knochenzementpulvers in die Kartusche; gegebenenfalls Abfließenlassen des PMMA-Knochenzementpulvers aus dem trichterförmigen Auslass und aus dem Einleitungsrohr in die Kartusche;
Anordnen der Vorrichtung, so dass die Monomerflüssigkeit aus dem Behälter durch freien Gravitationsfluss über den trichterförmigen Auslass und das Einleitungsrohr in die Kartusche überführbar ist;
Überführen der Monomerflüssigkeit aus dem Behälter in die Kartusche durch freien Gravitationsfluss;
Mischen der Monomerflüssigkeit mit dem PMMA-Knochenzementpulver in der Kartusche und dadurch Erhalten eines Knochenzementteigs.

Ein weiterer Aspekt betrifft die Verwendung einer Vorrichtung nach einer der vorangehenden Ausführungsformen oder des vorangehend genannten Verfahrens zur Herstellung von Knochenzementteig, insbesondere PMMA-Knochenzementteig.

### KURZE BESCHREIBUNG DER ZEICHNUNGEN

**Figur 1** zeigt eine Ausführungsform einer erfindungsgemäßen Vorrichtung.
**Figur 2** zeigt einen Ausschnitt einer erfindungsgemäßen Vorrichtung.
**Figur 3** zeigt eine erfindungsgemäße Vorrichtung, welche eine Glasampulle mit Monomerflüssigkeit und PMMA-Knochenzementpulver enthält.
**Figur 4** zeigt eine Vorrichtung, in welcher Monomerflüssigkeit durch einen trichterförmigen Auslass und ein Einleitungsrohr in die Kartusche der Vorrichtung gelangt.
**Figur 5** zeigt eine Vorrichtung, in welcher eine Monomerflüssigkeit und ein PMMA-Knochenzementpulver mithilfe eines Mischstabs zu einem Knochenzementteig gemischt werden.
**Figur 6** zeigt eine Vorrichtung, in welcher mithilfe einer Gewindestange und eines Kolbens Knochenzementteig aus der Vorrichtung durch ein Austragsrohr abgegeben werden.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse, Vorrichtungen, Kits und Verwendungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Eine erste Ausführungsform betrifft eine Vorrichtung zum Mischen von Knochenzement, welche eine Kartusche zur Aufnahme eines PMMA-Knochenzementpulvers; einen Behälter zur Aufnahme einer Monomerflüssigkeit, wobei der Behälter fluidleitend mit der Kartusche verbunden ist; ein Einleitungsrohr; und einen trichterförmigen Auslass aufweist, wobei die Vorrichtung ausgestaltet und eingerichtet ist, eine Monomerflüssigkeit aus dem Behälter durch freien Gravitationsfluss über den trichterförmigen Auslass und das Einleitungsrohr in die Kartusche zu überführen.

Eine weitere Ausführungsform betrifft eine Vorrichtung zum Mischen von Knochenzement, aufweisend
eine Kartusche zur Aufnahme eines PMMA-Knochenzementpulvers;
einen Behälter zur Aufnahme einer Monomerflüssigkeit, wobei der Behälter fluidleitend mit der Kartusche verbunden ist;
einen an einem proximalen Ende der Kartusche angeordneten Kartuschenkopf, wobei der Kartuschenkopf eine Durchführung aufweist;
ein Einleitungsrohr, wobei das Einleitungsrohr eine Längsachse aufweist;
und einen trichterförmigen Auslass, wobei der trichterförmige Auslass eine Trichterinnenfläche aufweist, die zumindest teilweise in einem Winkel von nicht mehr als 60° zur Längsachse des Einleitungsrohrs angeordnet ist;
wobei die Vorrichtung ausgestaltet und eingerichtet ist, eine Monomerflüssigkeit aus dem Behälter durch freien Gravitationsfluss über den trichterförmigen Auslass und das Einleitungsrohr in die Kartusche zu überführen.

Die Vorrichtung enthält eine Kartusche, die zur Aufnahme eines PMMA-Knochenzementpulvers geeignet ist. Die Kartusche kann beispielsweise ein im wesentlichen zylinderförmiges Gehäuse aufweisen. Das Gehäuse kann beispielsweise einen Kunststoff aufweisen oder aus Kunststoff bestehen. Die Kartusche kann ein PMMA-Knochenzementpulver enthalten. Ein PMMA-Knochenzementpulver ist eine pulverförmige Komponente, welche zur Herstellung eines härtbaren, d. h. polymerisierbaren, Knochenzementteigs dienen kann. Ein PMMA-Knochenzementpulver wird hierin auch als Pulverkomponente eines Knochenzements bezeichnet. Ein PMMA-Knochenzementpulver kann ein oder mehrere Polymere aufweisen. Solche Polymere können durch Polymerisation auf Basis von Methylmethacrylat und Comonomeren, wie z.B. Styren oder Methylacrylat, hergestellt werden.

Durch Vermischung eines PMMA-Knochenzementpulvers mit einer Monomerflüssigkeit kann ein Knochenzementteig gebildet werden, der zu einem PMMA-Knochenzement aushärten kann. Eine Monomerflüssigkeit kann beispielsweise das Monomer Methylmethacrylat und einen darin gelösten Aktivator aufweisen. Ein Beispiel für einen Aktivator ist N,N-Dimethyl-p-toluidin.

Demnach stellen ein PMMA-Knochenzementpulver und eine Monomerflüssigkeit jeweils Komponenten dar, die miteinander gemischt werden können, um einen Knochenzementteig zu bilden, der zu einem Knochenzement aushärtbar ist. Ein PMMA-Knochenzementpulver und eine Monomerflüssigkeit stellen somit jeweils Vorläufersubstanzen (d.h. Komponenten) zur Herstellung eines Knochenzements, insbesondere eines PMMA-Knochenzements, dar.

Die Kartusche weist ein proximales Ende auf, an dem einen Kartuschenkopf angeordnet ist. Der Kartuschenkopf kann lösbar mit der Kartusche verbindbar sein, z.B. mithilfe eines Gewindes. Der Kartuschenkopf kann als Verschluss der Kartusche dienen. Der Kartuschenkopf kann weiterhin eine Durchführung aufweisen. Die Durchführung kann zur Aufnahme eines Mischstabs eingerichtet sein. Mithilfe eines Mischstabs können ein PMMA-Knochenzementpulver und eine Monomerflüssigkeit innerhalb der Kartusche gemischt werden. Der Mischstab kann Flügel aufweisen, um das Mischen dieser beiden Komponenten zu erleichtern.

Der Kartuschenkopf kann weiterhin eine Öffnung zum Anschluss eines Austragsrohrs aufweisen. Ein solches Austragsrohr kann zur Abgabe von Knochenzementteig aus der Vorrichtung dienen. Eine Öffnung zum Anschluss eines Austragsrohrs kann auch an einer anderen Position der Kartusche angeordnet sein. Die Öffnung kann reversibel verschließbar sein, beispielsweise durch einen Gewindestopfen. Die Öffnung kann ein Gewinde aufweisen, das zur Herstellung einer reversiblen Verbindung mit einem Verschluss und/oder einem Austragsrohr eingerichtet ist.

In einer Ausführungsform weist die Vorrichtung einen Verschluss auf, der diese Öffnung verschließt, bevorzugt reversibel verschließt.

Alternativ oder zusätzlich kann der Kartuschenkopf eine Öffnung aufweisen, um das Einleitungsrohr aufzunehmen. Das Einleitungsrohr kann durch die Öffnung in die Kartusche einführbar sein, um eine fluidleitende Verbindung zwischen Behälter und Kartusche herzustellen.

In einer Ausführungsform weist die Vorrichtung ein Mittel zur Fixierung, bevorzugt lösbaren Fixierung, des Einleitungsrohrs an der Kartusche auf. In einer Ausführungsform ist der Verschluss eingerichtet, in das Mittel zur Fixierung einzugreifen.

Die Öffnung zur Aufnahme des Einleitungsrohrs kann beispielsweise durch einen Schiebeverschluss verschließbar sein. Der Schiebeverschluss kann eingerichtet sein, in eine Kerbe des Einleitungsrohrs einzugreifen.

Eine Öffnung in dem Kartuschenkopf kann eingerichtet sein, wahlweise ein Austragsrohr oder das Einleitungsrohr aufzunehmen.

Die Vorrichtung weist weiterhin einen Behälter zur Aufnahme einer Monomerflüssigkeit auf. Der Behälter kann eine Halterung für eine Glasampulle aufweisen. Eine Monomerflüssigkeit kann in einer Glasampulle bereitgestellt werden, und in einer solchen Halterung des Behälters aufnehmbar sein. Hierdurch können unerwünschte chemische Reaktionen der Monomerflüssigkeit während der Lagerung verhindert werden. Der Behälter kann auch eine Halterung für einen Folienbeutel aufweisen. Eine Monomerflüssigkeit kann auch in einem versiegelten Folienbeutel bereitgestellt werden.

Die Halterung kann bevorzugt in einem Winkel von 21° bis 50° zur Längsachse der Kartusche oder zur Längsachse des trichterförmigen Auslass angeordnet sein. Dies kann den Transfer der Monomerflüssigkeit in die Kartusche verbessern.

Die Vorrichtung weist weiterhin einen trichterförmigen Auslass und ein Einleitungsrohr auf. Mithilfe des Auslasses und des Einleitungsrohrs kann eine Monomerflüssigkeit aus dem Behälter in die Kartusche überführt werden, um ein Mischen der Monomerflüssigkeit mit dem PMMA-Knochenzementpulver innerhalb der Kartusche zu ermöglichen. Der Auslass kann beispielsweise als trichterförmige Verjüngung des Behälters ausgestaltet sein, wobei diese Verjüngung unmittelbar mit dem Einleitungsrohr verbunden sein kann. Das Einleitungsrohr kann an derjenigen Seite, welche gegenüber dem trichterförmigen Auslass angeordnet ist, mit der Kartusche verbunden sein. Somit kann eine Monomerflüssigkeit über den trichterförmigen Auslass und das Einleitungsrohr durch freien Gravitationsfluss in die Kartusche gelangen. In einigen Ausführungsformen ist das Einleitungsrohr reversibel lösbar mit der Kartusche verbindbar.

"Freier Gravitationsfluss" bedeutet hierin, dass eine Monomerflüssigkeit allein durch die Wirkung der Schwerkraft aus dem Behälter in die Kartusche gelangen kann, ohne dass beispielsweise ein Druckunterschied zwischen dem Behälter und der Kartusche für die Überführung der Monomerflüssigkeit erforderlich ist. Bevorzugt erlauben die hierin beschriebenen Vorrichtungen eine vollständige Überführung der Monomerflüssigkeit aus dem Behälter in die Kartusche durch freien Gravitationsfluss innerhalb von 20 Sekunden.

Der trichterförmige Auslass kann beispielsweise eine Form aufweisen, die durch eine lineare oder rotierende Extrusion eines Dreiecks definiert ist. Der trichterförmige Auslass kann somit beispielsweise die Form eines Kegels oder Halbkegels aufweisen. Der Auslass kann auch die Form einer Pyramide oder eines Pyramidenstumpfs aufweisen. "Trichterförmig" bedeutet hierin somit, dass sich der Innenraum des Auslasses in Richtung des Einleitungsrohrs zumindest abschnittsweise in gleichförmiger Weise verjüngt. Der trichterförmige Auslass weist eine Trichterinnenfläche auf. Diese Trichterinnenfläche ist in einem Winkel zu einer Längsachse des Einleitungsrohrs angeordnet, welcher bevorzugt höchstens 60° beträgt. Dies bewirkt, dass der trichterförmige Auslass ausreichend steil ausgestaltet ist, um ein freies Abfließen der Monomerflüssigkeit in die Kartusche zu gewährleisten.

Das Einleitungsrohr ist bevorzugt zylinderförmig. In einer Ausführungsform ist die Innenwand des Einleitungsrohrs zylinderförmig.

Der trichterförmige Auslass kann einen transparenten Bereich aufweisen. Dies kann es einem Benutzer der Vorrichtung erlauben, das vollständige Abfließen von Monomerflüssigkeit und/oder PMMA-Knochenzementpulver aus dem Auslass von außen visuell zu überprüfen.

Das Einleitungsrohr weist bevorzugt einen Innendurchmesser i von 4,0 mm bis 10,0 mm auf. Weiter bevorzugt kann der Innendurchmesser i des Einleitungsrohrs 4,0 mm bis 8,0 mm; 5,0 mm bis 8,0 mm; oder 4,0 mm bis 6,0 mm betragen. Beispielsweise beträgt der Innendurchmesser i mindestens 4,0 mm. Durchmesser von weniger als 4 mm können zur Ausbildung eines Meniskus in der Monomerflüssigkeit führen, der ein freies Abfließen der Monomerflüssigkeit verhindern kann. Zudem bilden sich bei Durchmessern von weniger als 4 mm häufig Pulverbrücken im PMMA-Knochenzementpulver.

In einer Ausführungsform weist das Einleitungsrohr eine Länge L und einen Innendurchmesser i auf. Die Länge L ist der kürzeste Abstand der beiden Enden des Einleitungsrohrs. Die Enden des Einleitungsrohrs können als diejenigen Punkte definiert sein, an denen das Einleitungsrohr mit dem trichterförmigen Auslass bzw. der Kartusche verbunden ist. Bevorzugt liegt das Verhältnis L : i von Länge L zu Innendurchmesser i des Einleitungsrohrs in einem Bereich von "L : i = 1 : 10" bis "L : i = 1 : 3,5". Dieser Bereich ist günstig zur Gewährleistung eines Ausfließens einer Monomerflüssigkeit aus dem Einleitungsrohr und des gleichzeitigen Aufsteigens von Luft in das Einleitungsrohr, um einen Druckausleich in der Vorrichtung zu erlauben.

Die Länge L des Einleitungsrohrs ist bevorzugt so gewählt, dass während der Verwendung der Vorrichtung das Einleitungsrohr keinen Kontakt zu einem PMMA-Knochenzementpulver in der Kartusche hat. Dadurch kann ein Verkleben des PMMA-Knochenzementpulvers während des Transfers von Monomerflüssigkeit verhindert werden. Dies bezieht sich insbesondere auf eine "aufrechte" Orientierung der Vorrichtung, welche einen freien Gravitationsfluss von Monomerflüssigkeit "nach unten" (d.h. in Schwerkraftrichtung) in die Kartusche zu ermöglicht. In einer solchen Orientierung zeigen die Längsachse des Einleitungsrohrs und deren Öffnung, die in Richtung der Kartusche angeordnet ist, "nach unten" (d.h. in Schwerkraftrichtung). Beispielsweise erstreckt sich das Einleitungsrohr nur zu 10%, zu 20% oder zu höchstens 30% der Kartuschenlänge in die Kartusche hinein. Die Kartuschenlänge ist hierbei als die Länge der Kartusche entlang der Längsachse der Kartusche definiert.

In einer Ausführungsform ist der trichterförmige Auslass ausgestaltet und angeordnet, ein Abfließen eines PMMA-Knochenzementpulvers durch freien Gravitationsfluss in die Kartusche zu ermöglichen. Auch wenn das PMMA-Knochenzementpulver in der Kartusche angeordnet ist, kann beispielsweise beim Transport der Vorrichtung PMMA-Knochenzementpulver teilweise durch das Einleitungsrohr in den Auslass gelangen. Um ein unerwünschtes Vermischen des PMMA-Knochenzementpulver mit Monomerflüssigkeit zu verhindern, kann der Auslass derart ausgestaltet und angeordnet sein, dass in einem solchen Fall das PMMA-Knochenzementpulver selbsttätig in die Kartusche zurückfließen kann. Hierfür kann insbesondere eine ausreichend steile Ausgestaltung und Anordnung des trichterförmigen Auslass von Bedeutung sein, gegebenenfalls in Verbindung mit dem Einleitungsrohr, wie hierin beschrieben. Dadurch kann ein Verkleben des PMMA-Knochenzementpulvers in dem Auslass oder in dem Einleitungsrohr während des Transfers von Monomerflüssigkeit verhindert werden.

In einer Ausführungsform weist die Vorrichtung weiterhin ein Mittel zur Eliminierung von PMMA-Knochenzementpulverbrücken in dem Auslass auf. Das Mittel zur Eliminierung von PMMA-Knochenzementpulverbrücken kann zum Beispiel eine Kugel sein. Bevorzugt kann die Kugel beweglich in dem trichterförmigen Auslass angeordnet sein. Die Kugel kann durch Bewegung im Innenraum des Auslasses Knochenzementpulverbrücken destabilisieren, sodass das PMMA-Knochenzementpulver rieselfähig wird und durch Gravitationsfluss aus dem Auslass in die Kartusche abgegeben werden kann. In ähnlicher Weise können andere geeignete Mittel eingesetzt werden, die Pulverbrücken des PMMA-Knochenzementpulvers destabilisieren, oder deren Aufbau verhindern. Beispielsweise können netzförmige oder stabförmige Strukturen in dem trichterförmigen Auslass angeordnet sein, die wie ein Sieb oder Rechen wirken, um Pulverbrücken zu vermeiden. Bevorzugte Mittel zur Eliminierung von PMMA-Knochenzementpulverbrücken sind solche, die das Abfließen von PMMA-Knochenzementpulver oder Monomerflüssigkeit nicht wesentlich verlangsamen, wie z.B. eine Kugel. Die Kugel weist bevorzugt einen Durchmesser auf, der größer als der Innendurchmesser des Einleitungsrohrs ist, beispielsweise mindestens 1,5-fach so groß wie der Innendurchmesser des Einleitungsrohrs. Hierdurch kann ein unerwünschter Verschluss des Einleitungsrohrs durch die Kugel vermieden werden.

Die Kugel hat bevorzugt eine Dichte von mindestens 1,0 g/cm³. Die Kugel kann ein Material aufweisen, das ausgewählt ist aus der Gruppe bestehend aus Glas, Keramik, Metall, Metalloxid, Kunststoff, und Kombinationen davon. Bevorzugte Keramiken sind Korund (Al₂O₃) oder Porzellan. Porzellan ist eine keramische Zusammensetzung, welche Kaolin, Quarz und Feldspat aufweist. Bevorzugte Metalle sind Edelstahl oder Tantal. Beispiele für Edelstahl sind die Legierungen 316L, 301 und 304.

In einigen Ausführungsformen bildet die Trichterinnenfläche des trichterförmigen Auslass über mindestens 50% ihrer gesamten Fläche einen Winkel von nicht mehr als 60° zur Längsachse der Einleitungsrohrs. Auf diese Weise wird eine steile Geometrie erreicht, die ein zuverlässiges Abfließen von Monomerflüssigkeit oder PMMA-Knochenzementpulver aus dem Auslass und über das Einleitungsrohr in die Kartusche per Gravitationsfluss ermöglicht.

In einigen Ausführungsformen bildet die Trichterinnenfläche des trichterförmigen Auslass über mindestens 50% ihrer gesamten Fläche einen Winkel von nicht mehr als 55°, weiter bevorzugt einen Winkel von 21° bis 50°, zur Längsachse der Einleitungsrohrs.

In einigen Ausführungsformen bildet die Trichterinnenfläche des trichterförmigen Auslass über mindestens 50% ihrer gesamten Fläche einen Winkel von 21° bis 60° zur Längsachse der Einleitungsrohrs.

In einigen Ausführungsformen weist die Kartusche ein PMMA-Knochenzementpulver auf. Das PMMA-Knochenzementpulver ist bevorzugt in einem Innenraum der Kartusche angeordnet.

In einigen Ausführungsformen weist der Behälter eine Monomerflüssigkeit auf. Die Monomerflüssigkeit ist bevorzugt in einem Innenraum des Behälters angeordnet. Die Monomerflüssigkeit kann in einer Glasampulle oder in einem versiegelten Folienbeutel innerhalb des Behälters angeordnet sein.

In einigen Ausführungsformen weist der trichterförmige Auslass ein verjüngtes Ende auf. Das verjüngte Ende kann unmittelbar fluidleitend mit dem Einleitungsrohr verbindbar sein oder verbunden sein. Der trichterförmige Auslass kann an einem verjüngten Ende des Auslasses unmittelbar in das Einleitungsrohr übergehen. Durch einen solchen direkten Übergang kann ein ungehindertes Abfließen von PMMA-Knochenzementpulver aus dem Auslass über das Einleitungsrohr und in die Kartusche erzielt werden.

In einigen Ausführungsformen weist der Behälter weiterhin einen für eine Monomerflüssigkeit durchlässigen Filter auf. Der Filter ist bevorzugt angeordnet und eingerichtet, ein Eindringen von Glassplittern aus dem Behälter in die Kartusche zu verhindern. Die Vorrichtung kann zur Aufnahme einer Glasampulle mit Monomerflüssigkeit eingerichtet sein. Die Vorrichtung kann weiterhin zum Aufbrechen einer Glasampulle mit Monomerflüssigkeit eingerichtet sein. Die Glasampulle kann insbesondere innerhalb des Behälters aufnehmbar und aufbrechbar sein. Der Behälter kann hierzu eine Aufnahme für eine Glasampulle aufweisen. Der Behälter kann ein Mittel zum Aufbrechen einer Glasampulle aufweisen. Beispielsweise kann der Behälter einen Vorsprung aufweisen, der zum Abbrechen des Kopfs einer Glasampulle angeordnet und eingerichtet ist.

Der Filter kann eingerichtet sein, Bruchstücke einer Glasampulle in dem Behälter zurückzuhalten. Hierdurch kann vermieden werden, dass Glassplitter in die Kartusche gelangen. Hierdurch kann eine Verunreinigung von Knochenzementteig mit Glassplittern vermieden werden. Bevorzugt kann der Filter an einer Seite des Filters eine Oberfläche von mind. 5 cm², weiter bevorzugt mind. 10 cm², noch weiter bevorzugt 15 cm² aufweisen. Diese Fläche entspricht derjenigen Fläche, die sich geometrisch unmittelbar aus dem im Fachgebiet geläufigen "Filterdurchmesser" ableitet.

Der Filter kann bevorzugt an einer Seite des Auslass angeordnet sein, die dem Einleitungsrohr gegenüber liegt.

In einigen Ausführungsformen bilden ein Innenraum des trichterförmigen Auslass und ein Innenraum der Kartusche gemeinsam einen kontinuierlichen Hohlraum. Dies bedeutet, dass die Innenräume von Auslass und Kartusche derart miteinander verbunden sind, dass PMMA-Knochenzementpulver ungehindert aus dem Auslass in die Kartusche fließen kann.

In einigen Ausführungsformen ist der trichterförmige Auslass ausgestaltet und angeordnet, die Ausbildung eines Meniskus zu verhindern, wenn eine Monomerflüssigkeit aus dem Behälter durch freien Gravitationsfluss über den trichterförmigen Auslass in die Kartusche überführt wird. Wie hierin andernorts ausführlicher erläutert, kann durch die geeignete Wahl der hierin beschriebenen Parameter, insbesondere des Winkels zwischen Trichterinnenfläche und Längsachse des Einleitungsrohrs, dem Innendurchmesser des Einleitungsrohrs, und der Länge des Einleitungsrohrs, ein ungehindertes Abfließen von Monomerflüssigkeit erreicht werden. Dieselben Parameter gewährleisten auch ein ungehindertes Abfließen von PMMA-Knochenzementpulver.

In einigen Ausführungsformen weist die Vorrichtung weiterhin einen Kolben zur Abgabe von Knochenzement aus der Vorrichtung auf.

Bevorzugt ist der Kolben zumindest teilweise in der Kartusche angeordnet und begrenzt die Kartusche nach außen. Weiter bevorzugt weist der Kolben bevorzugt eine lösbare Verriegelung auf. Diese Verriegelung kann beispielsweise als Stift, Lasche oder Schnapphaken ausgestaltet sein. Auch andere kraftschlüssige oder formschlüssige Verbindungen sind hierbei möglich. Hierdurch kann der Kolben lösbar mit der Kartusche verbindbar sein. Der Kolben kann beweglich in der Kartusche angeordnet sein, so dass er den Austrag von Knochenzementteig aus der Kartusche ermöglichen kann. Das Merkmal "beweglich in der Kartusche angeordnet" schließt das Vorhandensein einer oben beschriebenen Verriegelung des Kolbens nicht aus. Somit bezieht sich der Ausdruck "beweglich in der Kartusche angeordnet" gegebenenfalls auch auf eine Vorrichtung, bei welcher der Kolben nur in einem Zustand der Vorrichtung beweglich ist, in der eine solche Verriegelung gelöst ist.

In einigen Ausführungsformen weist die Vorrichtung einen Mischstab auf. Der Mischstab ist bevorzugt zum Mischen von Monomerflüssigkeit und PMMA-Knochenzementpulver eingerichtet. Der Mischstab kann Flügel aufweisen. Die Flügel können eine homogene Mischung von Monomerflüssigkeit und PMMA-Knochenzementpulver erleichtern. Die Flügel sind bevorzugt an einem Ende des Mischstabs angeordnet, welches innerhalb der Kartusche angeordnet ist. Der Mischstab kann einen, zwei oder mehr als zwei Flügel aufweisen. Der Mischstab kann an einem Ende des Mischstabs, welches bevorzugt außerhalb der Kartusche angeordnet ist, einen Griff aufweisen.

Der Mischstab kann ein Austragsrohr aufweisen. Hierbei kann das Austragsrohr zur Abgabe eines in der Vorrichtung gemischten Knochenzementteigs (d.h. eines Knochenzementteigs, der aus Monomerflüssigkeit und PMMA-Knochenzementpulver gemischt wurde) ausgebildet und eingerichtet sein. Bevorzugt ist das Austragsrohr verschließbar. Hierdurch kann verhindert werden, dass Knochenzementteig während des Mischens unerwünscht aus der Kartusche austritt. Das Austragsrohr kann als Hohlraum innerhalb des Mischstabs ausgebildet sein. In einigen Ausführungsformen ist das Austragsrohr durch einen lösbaren Kern verschließbar. Demnach kann der Mischstab einen lösbaren, stabförmigen Kern aufweisen, beispielsweise einen Metallkern. Der Kern kann auch die Festigkeit des Mischstabs verbessern.

Der Mischstab kann eine Sollbruchstelle aufweisen, um den Mischstab nach dem Mischvorgang abzubrechen. Die Sollbruchstelle ist bevorzugt außerhalb der Kartusche angeordnet, so dass der Mischstab ohne ein Öffnen der Kartusche von außen abgebrochen werden kann.

In einigen Ausführungsformen weist der Behälter weiterhin einen Träger zur Aufnahme einer Ampulle auf. Solche Ampullen können insbesondere Glasampullen sein, in denen Monomerflüssigkeit kommerziell erhältlich ist. Der Träger ist bevorzugt angeordnet und eingerichtet, das Aufbrechen einer Ampulle und das Abfließen von Monomerflüssigkeit aus der Ampulle durch den Auslass und das Einleitungsrohr durch freien Gravitationsfluss zu erlauben.

In einigen Ausführungsformen weist der Behälter weiterhin ein Mittel zur Öffnung eines Folienbeutels oder einer Glasampulle innerhalb der Vorrichtung auf. Ein Beispiel für ein Mittel zur Öffnung eines Folienbeutels ist ein Dorn, welcher zum Durchstechen eines Folienbeutels geeignet ist. Ein solcher Dorn kann beispielsweise aus einem starren Kunststoff oder aus Metall ausgebildet sein. Ein Beispiel für ein Mittel zur Öffnung einer Glasampulle ist ein Vorsprung, gegen den der Kopf einer Glasampulle gedrückt werden kann, um sie aufzubrechen. Ein Mittel zur Öffnung einer Glasampulle kann auch ein flexibles Gehäuseteil aufweisen, so dass die Glasampulle von außen mit der Hand gebrochen werden kann, indem das flexible Gehäuseteil verformt wird. Ein Mittel zur Öffnung einer Glasampulle kann auch ein beweglich angeordnetes Element wie z.B. einen Kolben aufweisen, welches die Glasampulle gegen ein hartes Gehäuseteil, z.B. einen Vorsprung, drücken und dadurch aufbrechen kann. Mittel zur Öffnung eines Folienbeutels oder einer Glasampulle sind weiterhin in DE19532015A1, WO9718031A1, EP2404864B1 und WO2010012114A1 offenbart.

In einigen Ausführungsformen ist die Vorrichtung ausgebildet und eingerichtet, eine Entlüftung der Kartusche durch den trichterförmigen Auslass zu ermöglichen, während eine Monomerflüssigkeit aus dem Behälter durch freien Gravitationsfluss über den trichterförmigen Auslass und das Einleitungsrohr in die Kartusche überführt wird. Dies bedeutet, dass durch den trichterförmigen Auslass gleichzeitig Luft aus der Kartusche in den Behälter gelangen kann, während Monomerflüssigkeit aus dem Behälter in die Kartusche fließt.

Dies kann insbesondere durch die hierin beschriebenen Ausgestaltungen des trichterförmigen Auslass und des Einleitungsrohrs erzielt werden. Weiterhin kann der Behälter eine Belüftungsöffnung aufweisen. Die Belüftungsöffnung kann wahlweise als einfache Öffnung oder als Ventil ausgestaltet sein. Die Öffnung kann an der Innenseite durch ein gasdurchlässiges poröses Material abgedeckt sein, so dass das Eindringen von Partikeln von außen in den Behälter vermieden werden kann, und gleichzeitig ein Luftaustausch durch das poröse Material möglich ist. Das Ventil kann beispielsweise ausgestaltet sein, Luft nur in Richtung des Innenraums des Behälters passieren zu lassen. Hierdurch kann der Transfer der Monomerflüssigkeit in die Kartusche verbessert werden, wenn der Transfer durch einen Druckunterschied erfolgt. Die Vorrichtung kann in einigen Ausführungsformen einen Anschluss für eine Vakuumquelle aufweisen, um wahlweise einen Transfer der Monomerflüssigkeit per Druckunterschied zu erlauben. Ein solcher Anschluss kann beispielsweise am Kartuschenkopf angeordnet sein. In einigen Ausführungsformen ist die Vorrichtung eingerichtet, einen Transfer von Monomerflüssigkeit aus dem Behälter in die Kartusche wahlweise durch freien Gravitationsfluss oder durch einen Druckunterschied zu erlauben.

Ein weiterer Aspekt betrifft ein Kit, welches mehrere getrennte Komponenten der hierin beschriebenen Vorrichtungen enthält. Das Kit kann beispielsweise ein erstes Element zur Aufnahme einer Monomerflüssigkeit und ein davon getrenntes zweites Element zur Aufnahme eines PMMA-Knochenzementpulvers aufweisen. Das erste Element weist bevorzugt einen Behälter zur Aufnahme einer Monomerflüssigkeit, einen trichterförmigen Auslass und ein Einleitungsrohr auf. Das zweite Element weist bevorzugt eine Kartusche zur Aufnahme eines PMMA-Knochenzementpulvers auf. Das zweite Element kann einen an einem proximalen Ende der Kartusche angeordneten Kartuschenkopf aufweisen. Der Kartuschenkopf kann eine Öffnung aufweisen, um das Einleitungsrohr des ersten Elements aufzunehmen. Das erste Element kann mit dem zweiten Element verbindbar sein, bevorzugt lösbar verbindbar sein. Die Öffnung kann reversibel verschließbar sein, beispielsweise durch einen Schiebeverschluss. Der Schiebeverschluss kann eingerichtet sein, in eine Kerbe des Einleitungsrohrs einzugreifen. Das Kit kann eine Monomerflüssigkeit aufweisen. Das Kit kann ein PMMA-Knochenzementpulver aufweisen. Das Kit kann eine Monomerflüssigkeit und ein PMMA-Knochenzementpulver aufweisen.

Das erste Element kann eine Monomerflüssigkeit enthalten. Eine Monomerflüssigkeit kann als Teil des Kits auch in einem separaten Gefäß bereitgestellt werden, beispielsweise in einem Folienbeutel oder einer Glasampulle. Das zweite Element kann ein PMMA-Knochenzementpulver enthalten. Ein PMMA-Knochenzementpulver kann als Teil des Kits auch in einem separaten Gefäß bereitgestellt werden, beispielsweise in einem Folienbeutel oder in einer Kunststoffdose. In einigen Ausführungsformen enthält das erste Element eine Monomerflüssigkeit, und das zweite Element enthält ein PMMA-Knochenzementpulver. Solche Vorrichtungen werden auch als "Full-Prepacked"-Systeme bezeichnet. Bevorzugt werden Monomerflüssigkeit und PMMA-Knochenzementpulver in steriler Form bereitgestellt. Auch die Vorrichtung wird bevorzugt steril bereitgestellt.

Ein weiterer Aspekt betrifft ein Verfahren zur Herstellung eines Knochenzements, wobei das Verfahren die nachfolgenden Schritte aufweist,
Bereitstellen einer Vorrichtung nach einer der hierin beschriebenen Ausführungsformen; gegebenenfalls Einbringen einer Monomerflüssigkeit in den Behälter;
gegebenenfalls Einbringen eines PMMA-Knochenzementpulvers in die Kartusche; gegebenenfalls Abfließenlassen des PMMA-Knochenzementpulvers aus dem trichterförmigen Auslass und/oder aus dem Einleitungsrohr in die Kartusche;
Anordnen der Vorrichtung, so dass die Monomerflüssigkeit aus dem Behälter durch freien Gravitationsfluss über den trichterförmigen Auslass und das Einleitungsrohr in die Kartusche überführbar ist;
Überführen der Monomerflüssigkeit aus dem Behälter in die Kartusche durch freien Gravitationsfluss;
Mischen der Monomerflüssigkeit mit dem PMMA-Knochenzementpulver in der Kartusche und dadurch Erhalten eines Knochenzements.

Das Verfahren kann beispielsweise einen, mehrere oder alle der folgenden Schritte umfassen:
a) Bereitstellen einer Vorrichtung nach einer der hierin beschriebenen Ausführungsformen, wobei die Vorrichtung bevorzugt einen Behälter, einen trichterförmigen Auslass, eine Glasampulle mit Monomerflüssigkeit, ein Einleitungsrohr, eine Kartusche, ein PMMA-Knochenzementpulver, einen Kartuschenkopf, einen Schiebeverschluss, einen Mischstab mit Kern, eine Öffnung im Kartuschenkopf zur Verbindung mit dem Einleitungsrohr, einen Schiebeverschluss, einen Kolben und ein Austragsrohr aufweist;
b) Ausrichten der Vorrichtung, sodass ein freier Gravitationsfluss der Monomerflüssigkeit ermöglicht wird;
c) Abfließenlassen von gegebenenfalls in dem trichterförmigen Auslass vorhandenem PMMA-Knochenzementpulver in einen Innenraum der Kartusche,
d) Freisetzen von Monomerflüssigkeit in dem Behälter, beispielsweise durch Aufbrechen einer Glasampulle mit Monomerflüssigkeit,
e) Überführen der Monomerflüssigkeit aus dem Behälter in die Kartusche durch freien Gravitationsfluss, wobei bevorzugt gleichzeitig Luft aus der Kartusche durch das Einleitungsrohr in den trichterförmigen Auslass einströmt,
f) Entfernen des Einleitungsrohrs aus der Kartusche,
g) Verschließen der Kartusche, beispielsweise Verschließen der Öffnung im Kartuschenkopf durch Einschieben eines Schiebeverschlusses,
h) Mischen der Monomerflüssigkeit mit dem PMMA-Knochenzementpulver mit dem Mischstab und dadurch Erhalt eines Knochenzementteigs;
i) Herausziehen des Kernes des Mischstabs, sodass der Mischstab ein Austragsrohr bildet;
j) Auspressen des Knochenzementteigs durch Verschieben des Kolbens in Richtung des Kartuschenkopfs, und
k) Austritt des Knochenzementteigs aus der Vorrichtung.

Ein weiterer Aspekt betrifft die Verwendung einer Vorrichtung nach einer der vorangehenden Ausführungsformen oder des vorangehend genannten Verfahrens zur Herstellung von Knochenzement, insbesondere PMMA-Knochenzement. Hierbei kann eine Monomerflüssigkeit mit einem PMMA-Knochenzementpulver innerhalb der Vorrichtung gemischt werden, um einen Knochenzementteig zu bilden. Ein solcher Knochenzementteig kann aus der Vorrichtung abgegeben werden und an einem Zielort zu PMMA-Knochenzement aushärten.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

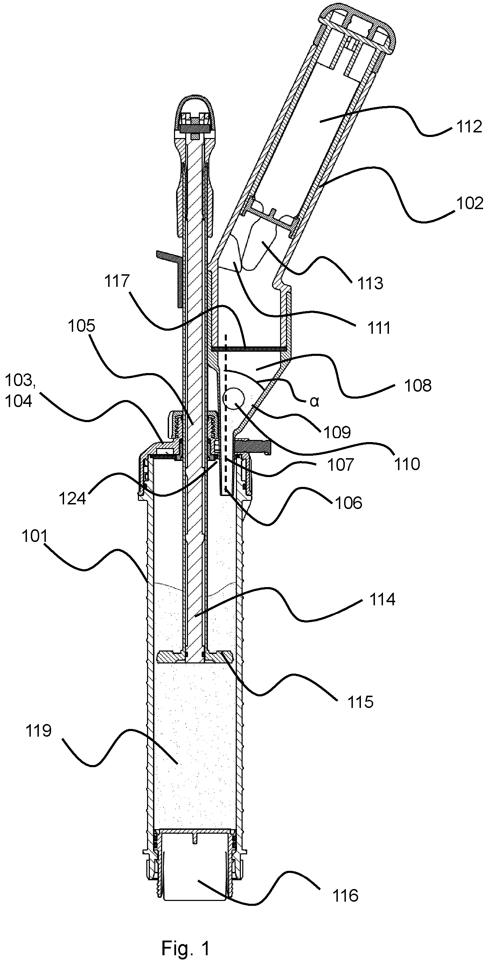

**Figur 1** zeigt beispielhaft eine Ausführungsform einer hierin beschriebenen Vorrichtung **100.** Die Vorrichtung **100** weist eine Kartusche **101** auf, die zur Aufnahme eines PMMA-Knochenzementpulvers **119** eingerichtet ist. An einem proximalen Ende der Kartusche **103** weist die Kartusche einen Kartuschenkopf **104** auf. Im hier gezeigten Beispiel ist der Kartuschenkopf **104** als Schraubdeckel ausgeführt. Der Kartuschenkopf **104** weist eine Durchführung **105** auf, die zur Aufnahme eines Mischstabs **114** eingerichtet ist. Der Mischstab **114** weist Flügel **115** auf, welche innerhalb der Kartusche **101** angeordnet sind. Der Mischstab kann zur Mischung einer Monomerflüssigkeit **118** mit PMMA-Knochenzementpulver **119** dienen, um daraus einen Knochenzementteig **123** zu bilden. Die Kartusche **101** weist weiterhin einen Kolben **116** auf, der zum Ausbringen von Knochenzementteig **123** aus der Vorrichtung eingerichtet ist. Die Vorrichtung weist weiterhin einen Behälter **102** auf, welcher zur Aufnahme einer Monomerflüssigkeit **118** eingerichtet ist. Der Behälter **102** ist eingerichtet, über einen trichterförmigen Auslass **108** und ein Einleitungsrohr **106** eine fluidleitende Verbindung mit der Kartusche **103** herzustellen. Hierzu kann das Einleitungsrohr **106** in eine Öffnung **124** des Kartuschenkopfs eingeschoben werden. Der trichterförmige Auslass **108** weist eine Trichterinnenfläche **109** auf, welche einen Winkel **α** mit einer Längsachse des Einleitungsrohrs **107** bildet, der höchstens 60° beträgt. Dies ermöglicht ein Abfließen von Monomerflüssigkeit oder PMMA-Knochenzementpulver aus dem Behälter **102** und dem trichterförmigen Auslass **108** durch das Einleitungsrohr **106** in die Kartusche **101,** ohne dass es hierzu einer mechanischen Kraftausübung oder eines Druckunterschieds bedarf. Vielmehr kann das Abfließen von Monomerflüssigkeit oder PMMA-Knochenzementpulver durch freien Gravitationsfluss erfolgen, d. h. durch die alleinige Wirkung der Schwerkraft. Hierzu kann die Vorrichtung mit dem Behälter **102** nach oben und mit der Kartusche **101** nach unten ausgerichtet sein, wie es hier gezeigt ist. Hierbei verläuft die Längsachse des Einleitungsrohrs **107** in Schwerkraftrichtung. Der Behälter **102** weist im hier gezeigten Beispiel weiterhin einen Filter **117** auf, welcher durchlässig für Monomerflüssigkeit ist, und am Übergang von Behälter **102** zu dem trichterförmigen Auslass **108** angeordnet ist. Der Filter **117** kann eingerichtet sein, dass Eindringen von Kontaminationen wie z.B. Glasbruchstücken in die Kartusche **101** zu verhindern. Der Behälter **102** weist im hier gezeigten Beispiel einen Träger für eine Ampulle **112,** beispielsweise eine Glasampulle, auf. Weiterhin weist der Behälter **102** hier einen Vorsprung **111** auf, um die Ampulle **112** aufzubrechen, indem der Ampullenkopf **113** der Ampulle **112** gegen den Vorsprung **111** gedrückt wird. Weiterhin ist in dem trichterförmigen Auslass **108** eine Kugel **110** angeordnet, die zur Eliminierung von PMMA-Knochenzementpulverbrücken eingerichtet ist. Durch Bewegung der Kugel **110** in dem Auslass **108,** die beispielsweise durch leichtes Schütteln der Vorrichtung bewirkt werden kann, können PMMA-Knochenzementpulverbrücken gelöst werden, sodass das PMMA-Knochenzementpulver aus dem Auslass in die Kartusche abfließen kann.

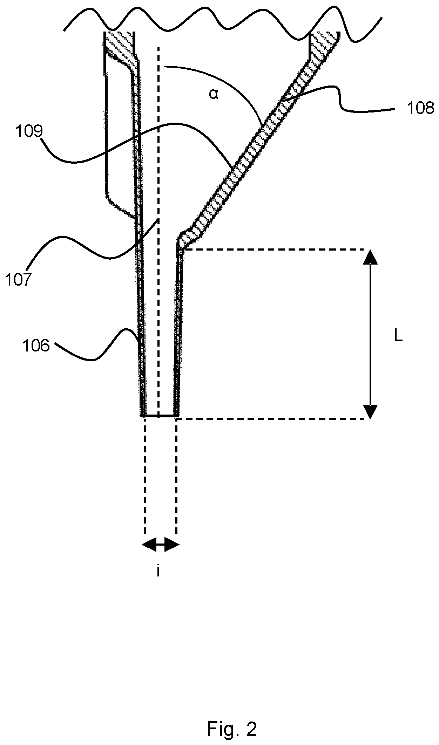

**Figur 2** zeigt beispielhaft einen Ausschnitt einer erfindungsgemäßen Vorrichtung. Ein trichterförmige Auslass **108** ist unmittelbar mit einem Einleitungsrohr **106** verbunden. Eine Trichterinnenfläche **109** bildet einen Winkel **α** mit einer Längsachse **107** des Einleitungsrohrs, der 60° oder weniger beträgt. Das Einleitungsrohr **106** weist eine Länge **L** und einen Durchmesser i auf. Das Verhältnis **L : i** von Länge L zu Innendurchmesser **i** liegt in einem Bereich von **"L : i = 1 : 10"** bis **"L : i = 1 : 3,5".** Der Innendurchmesser **i** beträgt 4,0 bis 10,0 cm.

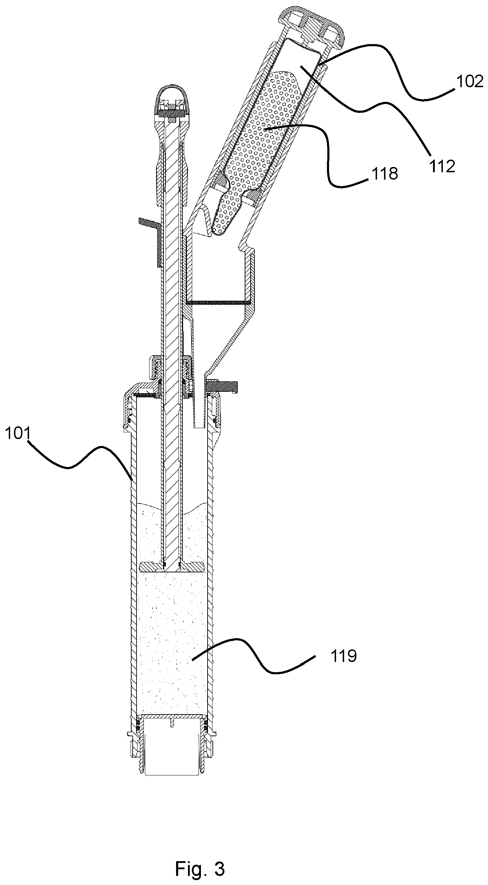

**Figur 3** zeigt beispielhaft eine Ausführungsform einer erfindungsgemäßen Vorrichtung. Im hier gezeigten Beispiel weist der Behälter **102** einen Träger für eine Glasampulle **112** auf, in der eine Monomerflüssigkeit **118** enthalten ist. In der Kartusche **101** ist ein PMMA-Knochenzementpulver **119** angeordnet. Das Einleitungsrohr **106** ragt nur so weit in die Kartusche **101** hinein, dass sie das PMMA-Knochenzementpulver **119** nicht berührt, wenn die Vorrichtung wie hier gezeigt aufrecht angeordnet ist.

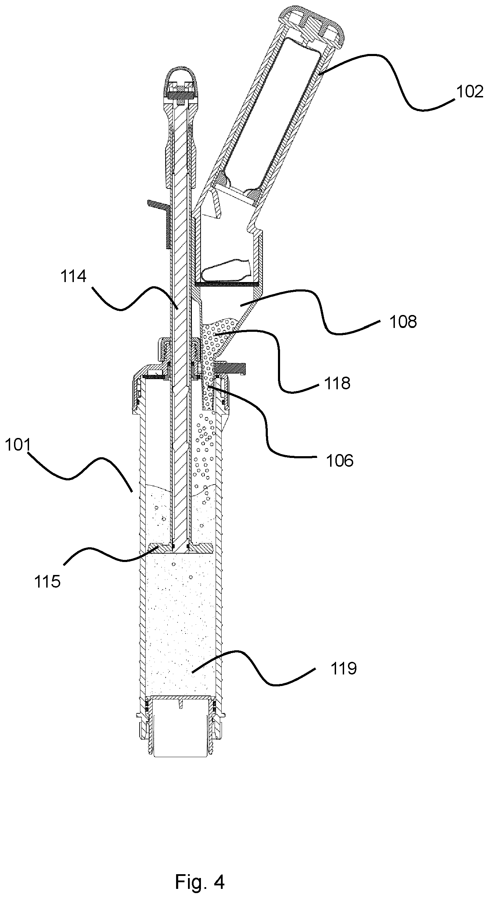

**Figur 4** zeigt beispielhaft eine Vorrichtung gemäß Figur 3, wobei die Monomerflüssigkeit **118** aus der Glasampulle freigesetzt ist. Die Monomerflüssigkeit **118** wird durch freien Gravitationsfluss aus dem Behälter **102** über den trichterförmigen Auslass **108** und das Einleitungsrohr **106** in die Kartusche **101** überführt. In der Kartusche gelangt die Monomerflüssigkeit **118** in Kontakt mit PMMA-Knochenzementpulver **119.** Zum Mischen von Monomerflüssigkeit **118** und PMMA-Knochenzementpulver **119** weist die Vorrichtung einen Mischstab **114** auf, der teilweise in die Kartusche **101** hineinragt, und Flügel **115** aufweist. Die Flügel **115** können ein homogenes und effektives Durchmischen von Monomerflüssigkeit **118** und PMMA-Knochenzementpulver **119** ermöglichen. Der Mischstab **114** weist weiterhin einen Metallkern auf, welcher einen Hohlraum im Inneren des Mischstabs verschließt. Durch Entfernen des Metallkerns kann der Hohlraum freigegeben werden, sodass der Hohlraum des Mischstabs zum Ausbringen von Knochenzementteig aus der Vorrichtung verwendbar ist. Weiterhin kann der Metallkern die Stabilität des Mischstabs verbessern, sodass eine verbesserte Kraftübertragung bei der Bewegung des Mischstabs erzielt werden kann.

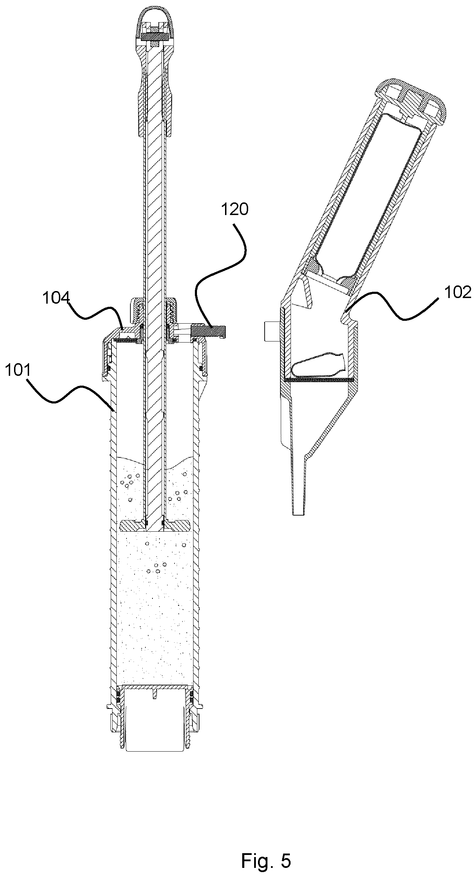

**Figur 5** zeigt beispielhaft eine Vorrichtung gemäß Figur 3 oder Figur 4, wobei der Behälter **102** nicht mehr fluidleitend mit der Kartusche **101** verbunden ist. In der hier gezeigten Konfiguration ist das Einleitungsrohr **106** aus der Öffnung **124** der Kartusche **101** herausgezogen. Die Öffnung **124** ist durch einen Verschluss **120** verschließbar. Der Verschluss **120** ist hier als Schiebeverschluss ausgeführt, der seitlich über die Öffnung **124** einschiebbar ist. Der Schiebeverschluss **124** ist im hier gezeigten Beispiel eingerichtet, in einem geschlossenen Zustand in den Kartuschenkopf **104** einzugreifen, sodass der Schiebeverschluss sich in dem geschlossenen Zustand nicht selbsttätig öffnen kann. Dies kann durch eine geeignete Verrastung an dem Kartuschenkopf erzielt werden. Durch die Trennung der Kartusche **101** von dem Behälter **102** kann eine verbesserte Handhabung der Kartusche **101** beim Mischen von Monomerflüssigkeit mit PMMA-Knochenzementpulver erzielt werden.

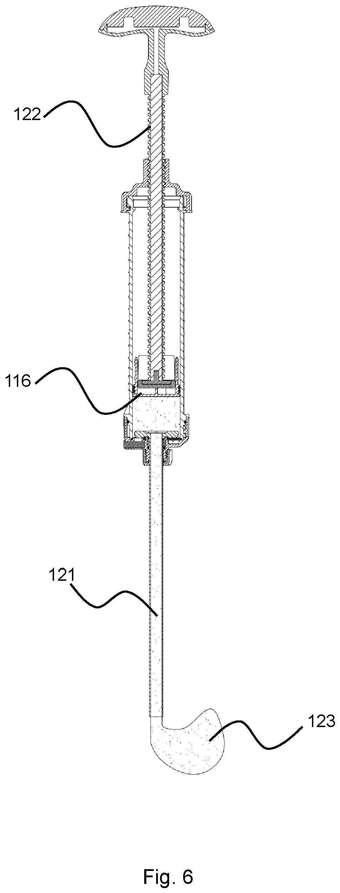

**Figur 6** zeigt beispielhaft eine erfindungsgemäße Vorrichtung, welche einen Knochenzementteig aus Monomerflüssigkeit und PMMA-Knochenzementpulver enthält. Die Vorrichtung weist in diesem Beispiel eine Gewindestange **122** auf, deren Gerwinde in den Kartuschenkopf **104** eingreift. Die Gewindestange **122** ist eingerichtet, einen in der Kartusche **101** beweglich angeordneten Kolben **116** zu bewegen, um Knochenzementteig **123** über ein Austragsrohr **121** aus der Vorrichtung abzugeben.

### BEZUGSZEICHENLISTE

- 100: Vorrichtung
- 101: Kartusche
- 102: Behälter
- 103: Proximales Ende der Kartusche
- 104: Kartuschenkopf
- 105: Durchführung
- 106: Einleitungsrohr
- 107: Längsachse des Einleitungsrohrs
- 108: Trichterförmiger Auslass
- 109: Trichterinnenfläche
- 110: Mittel zur Eliminierung von PMMA-Knochenzementpulverbrücken
- 111: Vorsprung
- 112: Ampulle
- 113: Ampullenkopf
- 114: Mischstab
- 115: Flügel
- 116: Kolben
- 117: Filter
- 118: Monomerflüssigkeit
- 119: PMMA-Knochenzementpulver
- 120: Verschluss
- 121: Austragsrohr
- 122: Gewindestange
- 123: Knochenzementteig
- 124: Öffnung

## Patentansprüche

1. Vorrichtung zum Mischen von Knochenzement, aufweisend
eine Kartusche (101) zur Aufnahme eines PMMA-Knochenzementpulvers;
einen Behälter (102) zur Aufnahme einer Monomerflüssigkeit (118), wobei der Behälter fluidleitend mit der Kartusche (101) verbunden ist;
einen an einem proximalen Ende (103) der Kartusche angeordneten Kartuschenkopf (104),
wobei der Kartuschenkopf eine Durchführung (105) aufweist;
ein Einleitungsrohr (106), wobei das Einleitungsrohr eine Längsachse (107) aufweist;
und einen trichterförmigen Auslass (108), wobei der trichterförmige Auslass eine Trichterinnenfläche (109) aufweist, die zumindest teilweise in einem Winkel (α) von nicht mehr als 60° zur Längsachse (107) des Einleitungsrohrs (106) angeordnet ist;
wobei die Vorrichtung ausgestaltet und eingerichtet ist, eine Monomerflüssigkeit aus dem Behälter durch freien Gravitationsfluss über den trichterförmigen Auslass und das Einleitungsrohr in die Kartusche zu überführen.

2. Vorrichtung nach Anspruch 1, wobei das Einleitungsrohr (106) einen Innendurchmesser i von 4,0 mm bis 10,0 mm aufweist.

3. Vorrichtung nach einem der vorangegangenen Ansprüche, wobei das Einleitungsrohr (106) eine Länge **L** und einen Innendurchmesser i aufweist, und wobei das Verhältnis **L : i** von Länge **L** zu Innendurchmesser **i** in einem Bereich von **"L : i = 1 : 10"** bis **"L : i = 1 : 3,5"** liegt.

4. Vorrichtung nach einem der vorangegangenen Ansprüche, wobei der trichterförmige Auslass (108) weiterhin ausgestaltet und angeordnet ist, ein Abfließen eines PMMA-Knochenzementpulvers durch freien Gravitationsfluss in die Kartusche (101) zu ermöglichen.

5. Vorrichtung nach einem der vorangegangenen Ansprüche, welche weiterhin ein Mittel (110) zur Eliminierung von PMMA-Knochenzementpulverbrücken in dem Auslass (108) aufweist, wobei das Mittel zur Eliminierung von PMMA-Knochenzementpulverbrücken bevorzugt eine Kugel ist, wobei die Kugel weiter bevorzugt beweglich in dem trichterförmigen Auslass angeordnet ist.

6. Vorrichtung nach einem der vorangegangenen Ansprüche, wobei die Trichterinnenfläche (109) über mindestens 50% ihrer gesamten Fläche einen Winkel α von nicht mehr als 60°, bevorzugt nicht mehr als 55°, weiter bevorzugt einen Winkel von 21° bis 50°, zur Längsachse (107) des Einleitungsrohrs (106) bildet.

7. Vorrichtung nach einem der vorangegangenen Ansprüche, wobei die Kartusche eine PMMA-Knochenzementpulver aufweist, und/oder wobei der Behälter eine Monomerflüssigkeit eines Knochenzements aufweist.

8. Vorrichtung nach einem der vorangegangenen Ansprüche, wobei der trichterförmige Auslass (108) ein verjüngtes Ende aufweist, welches unmittelbar fluidleitend mit dem Einleitungsrohr (106) verbunden ist.

9. Vorrichtung nach einem der vorangegangenen Ansprüche, wobei der Behälter weiterhin einen für eine Monomerflüssigkeit durchlässigen Filter (117) aufweist, wobei der Filter bevorzugt angeordnet und eingerichtet ist, ein Eindringen von Glassplittern aus dem Behälter in die Kartusche zu verhindern, und wobei der Filter an einer Seite des Filters bevorzugt eine Oberfläche von mind. 5 cm², weiter bevorzugt mind. 10 cm², noch weiter bevorzugt 15 cm² aufweist.

10. Vorrichtung nach einem der vorangegangenen Ansprüche, wobei ein Innenraum des trichterförmigen Auslass und ein Innenraum der Kartusche gemeinsam einen kontinuierlichen Hohlraum bilden.

11. Vorrichtung nach einem der vorangegangenen Ansprüche, wobei der trichterförmige Auslass ausgestaltet und angeordnet ist, die Ausbildung eines Meniskus zu verhindern, wenn eine Monomerflüssigkeit aus dem Behälter durch freien Gravitationsfluss über den trichterförmigen Auslass in die Kartusche überführt wird.

12. Vorrichtung nach einem der vorangegangenen Ansprüche, weiterhin aufweisend einen Kolben (116) zur Abgabe von Knochenzement aus der Vorrichtung, wobei der Kolben zumindest teilweise in der Kartusche angeordnet ist und die Kartusche nach außen begrenzt, und wobei der Kolben bevorzugt eine lösbare Verriegelung aufweist.

13. Vorrichtung nach einem der vorangegangenen Ansprüche, weiterhin aufweisend einen Mischstab (114), wobei der Mischstab bevorzugt ein verschließbares Austragsrohr aufweist, wobei das Austragsrohr bevorzugt ausgebildet und eingerichtet zur Abgabe eines in der Vorrichtung gemischten Knochenzementteigs aus der Vorrichtung ist.

14. Vorrichtung nach einem der vorangegangenen Ansprüche, wobei der Behälter weiterhin einen Träger zur Aufnahme einer Ampulle aufweist.

15. Vorrichtung nach einem der vorangegangenen Ansprüche, wobei der Behälter weiterhin ein Mittel (111) zur Öffnung eines Folienbeutels oder einer Glasampulle innerhalb der Vorrichtung aufweist.

16. Vorrichtung nach einem der vorangegangenen Ansprüche, wobei die Vorrichtung ausgebildet und eingerichtet ist, eine Entlüftung der Kartusche durch den trichterförmigen Auslass zu ermöglichen, während eine Monomerflüssigkeit aus dem Behälter durch freien Gravitationsfluss über den trichterförmigen Auslass und das Einleitungsrohr in die Kartusche überführt wird.

17. Verfahren zur Herstellung eines Knochenzementteigs, wobei das Verfahren die nachfolgenden Schritte aufweist,
Bereitstellen einer Vorrichtung nach einem der vorangehenden Ansprüche;
gegebenenfalls Einbringen einer Monomerflüssigkeit in den Behälter;
gegebenenfalls Einbringen eines PMMA-Knochenzementpulvers in die Kartusche;
gegebenenfalls Abfließenlassen des PMMA-Knochenzementpulvers aus dem trichterförmigen Auslass und aus dem Einleitungsrohr in die Kartusche;
Anordnen der Vorrichtung, so dass die Monomerflüssigkeit aus dem Behälter durch freien Gravitationsfluss über den trichterförmigen Auslass und das Einleitungsrohr in die Kartusche überführbar ist;
Überführen der Monomerflüssigkeit aus dem Behälter in die Kartusche durch freien Gravitationsfluss;
Mischen der Monomerflüssigkeit mit dem PMMA-Knochenzementpulver in der Kartusche und dadurch Erhalten eines Knochenzementteigs.

18. Verwendung einer Vorrichtung gemäß einem der Ansprüche 1 bis 16 oder eines Verfahrens gemäß Anspruch 17 zur Herstellung von Knochenzementteig.

## Geänderte Patentansprüche

### Geänderte Patentansprüche gemäss Regel 137(2) EPÜ.

1. Vorrichtung zum Mischen von Knochenzement, aufweisend
eine Kartusche (101) zur Aufnahme eines PMMA-Knochenzementpulvers;
einen Behälter (102) zur Aufnahme einer Monomerflüssigkeit (118), wobei der Behälter fluidleitend mit der Kartusche (101) verbunden ist;
einen an einem proximalen Ende (103) der Kartusche angeordneten Kartuschenkopf (104), wobei der Kartuschenkopf eine Durchführung (105) aufweist;
ein Einleitungsrohr (106), wobei das Einleitungsrohr eine Längsachse (107) aufweist;
und einen trichterförmigen Auslass (108), wobei der trichterförmige Auslass eine Trichterinnenfläche (109) aufweist, die zumindest teilweise in einem Winkel (α) von nicht mehr als 60° zur Längsachse (107) des Einleitungsrohrs (106) angeordnet ist;
wobei die Vorrichtung ausgestaltet und eingerichtet ist, eine Monomerflüssigkeit aus dem Behälter durch freien Gravitationsfluss über den trichterförmigen Auslass und das Einleitungsrohr in die Kartusche zu überführen wobei die Trichterinnenfläche (109) über mindestens 50% ihrer gesamten Fläche einen Winkel α von nicht mehr als 60°, bevorzugt nicht mehr als 55°, weiter bevorzugt einen Winkel von 21° bis 50°, zur Längsachse (107) des Einleitungsrohrs (106) bildet.

2. Vorrichtung nach Anspruch 1, wobei das Einleitungsrohr (106) einen Innendurchmesser i von 4,0 mm bis 10,0 mm aufweist.

3. Vorrichtung nach einem der vorangegangenen Ansprüche, wobei das Einleitungsrohr (106) eine Länge L und einen Innendurchmesser i aufweist, und wobei das Verhältnis L : i von Länge L zu Innendurchmesser i in einem Bereich von "L : i = 1 : 10" bis "L : i = 1 : 3,5" liegt.

4. Vorrichtung nach einem der vorangegangenen Ansprüche, wobei der trichterförmige Auslass (108) weiterhin ausgestaltet und angeordnet ist, ein Abfließen eines PMMA-Knochenzementpulvers durch freien Gravitationsfluss in die Kartusche (101) zu ermöglichen.

5. Vorrichtung nach einem der vorangegangenen Ansprüche, welche weiterhin ein Mittel (110) zur Eliminierung von PMMA-Knochenzementpulverbrücken in dem Auslass (108) aufweist, wobei das Mittel zur Eliminierung von PMMA-Knochenzementpulverbrücken bevorzugt eine Kugel ist, wobei die Kugel weiter bevorzugt beweglich in dem trichterförmigen Auslass angeordnet ist.

6. Vorrichtung nach einem der vorangegangenen Ansprüche, wobei die Kartusche eine PMMA-Knochenzementpulver aufweist, und/oder wobei der Behälter eine Monomerflüssigkeit eines Knochenzements aufweist.

7. Vorrichtung nach einem der vorangegangenen Ansprüche, wobei der trichterförmige Auslass (108) ein verjüngtes Ende aufweist, welches unmittelbar fluidleitend mit dem Einleitungsrohr (106) verbunden ist.

8. Vorrichtung nach einem der vorangegangenen Ansprüche, wobei der Behälter weiterhin einen für eine Monomerflüssigkeit durchlässigen Filter (117) aufweist, wobei der Filter bevorzugt angeordnet und eingerichtet ist, ein Eindringen von Glassplittern aus dem Behälter in die Kartusche zu verhindern, und wobei der Filter an einer Seite des Filters bevorzugt eine Oberfläche von mind. 5 cm², weiter bevorzugt mind. 10 cm², noch weiter bevorzugt 15 cm² aufweist.

9. Vorrichtung nach einem der vorangegangenen Ansprüche, wobei ein Innenraum des trichterförmigen Auslass und ein Innenraum der Kartusche gemeinsam einen kontinuierlichen Hohlraum bilden.

10. Vorrichtung nach einem der vorangegangenen Ansprüche, wobei der trichterförmige Auslass ausgestaltet und angeordnet ist, die Ausbildung eines Meniskus zu verhindern, wenn eine Monomerflüssigkeit aus dem Behälter durch freien Gravitationsfluss über den trichterförmigen Auslass in die Kartusche überführt wird.

11. Vorrichtung nach einem der vorangegangenen Ansprüche, weiterhin aufweisend einen Kolben (116) zur Abgabe von Knochenzement aus der Vorrichtung, wobei der Kolben zumindest teilweise in der Kartusche angeordnet ist und die Kartusche nach außen begrenzt, und wobei der Kolben bevorzugt eine lösbare Verriegelung aufweist.

12. Vorrichtung nach einem der vorangegangenen Ansprüche, weiterhin aufweisend einen Mischstab (114), wobei der Mischstab bevorzugt ein verschließbares Austragsrohr aufweist, wobei das Austragsrohr bevorzugt ausgebildet und eingerichtet zur Abgabe eines in der Vorrichtung gemischten Knochenzementteigs aus der Vorrichtung ist.

13. Vorrichtung nach einem der vorangegangenen Ansprüche, wobei der Behälter weiterhin einen Träger zur Aufnahme einer Ampulle aufweist.

14. Vorrichtung nach einem der vorangegangenen Ansprüche, wobei der Behälter weiterhin ein Mittel (111) zur Öffnung eines Folienbeutels oder einer Glasampulle innerhalb der Vorrichtung aufweist.

15. Vorrichtung nach einem der vorangegangenen Ansprüche, wobei die Vorrichtung ausgebildet und eingerichtet ist, eine Entlüftung der Kartusche durch den trichterförmigen Auslass zu ermöglichen, während eine Monomerflüssigkeit aus dem Behälter durch freien Gravitationsfluss über den trichterförmigen Auslass und das Einleitungsrohr in die Kartusche überführt wird.

16. Verfahren zur Herstellung eines Knochenzementteigs, wobei das Verfahren die nachfolgenden Schritte aufweist,
Bereitstellen einer Vorrichtung nach einem der vorangehenden Ansprüche;
gegebenenfalls Einbringen einer Monomerflüssigkeit in den Behälter;
gegebenenfalls Einbringen eines PMMA-Knochenzementpulvers in die Kartusche; gegebenenfalls Abfließenlassen des PMMA-Knochenzementpulvers aus dem trichterförmigen Auslass und aus dem Einleitungsrohr in die Kartusche;
Anordnen der Vorrichtung, so dass die Monomerflüssigkeit aus dem Behälter durch freien Gravitationsfluss über den trichterförmigen Auslass und das Einleitungsrohr in die Kartusche überführbar ist;
Überführen der Monomerflüssigkeit aus dem Behälter in die Kartusche durch freien Gravitationsfluss;
Mischen der Monomerflüssigkeit mit dem PMMA-Knochenzementpulver in der Kartusche und dadurch Erhalten eines Knochenzementteigs.

17. Verwendung einer Vorrichtung gemäß einem der Ansprüche 1 bis 15 oder eines Verfahrens gemäß Anspruch 16 zur Herstellung von Knochenzementteig.
